# EUROPEAN PATENT APPLICATION

(11) **EP 4 686 489 A1**
(43) Date of publication of application: **04.02.2026**
(21) Application number: 24192426.5
(22) Date of filing: 01.08.2024
(51) Int. Cl.: A61M 11/00, A61M 15/00

(54) **SOFT MIST INHALER**

(71) Applicant: Dunne, Stephen Terence, 4150-044 Porto (PT)
(72) Inventor: Dunne, Stephen Terence, 4150-044 Porto (PT)
(74) Representative: Tergau & Walkenhorst Intellectual Property GmbH

(57) **Abstract**

A soft mist inhaler (1), comprising a continuously pressurized collapsible container (6), a high-pressure pump (4) with a metering chamber (16), and an atomizer (18) connected to the outlet of the metering chamber (16), wherein the collapsible container (6) is connected to an inlet of the metering chamber (16) via a liquid passageway (22) to allow liquid flow from the collapsible container (6) to the metering chamber (16) during a priming stroke, should be designed high reliability in avoiding leakage via the nozzle in a particularly simple design. According to the invention this is achieved with a pressure-relief valve (34) provided in the connection between the metering chamber (16) and the atomizer (18), having an opening pressure of not more than 150% of the gauge pressure in the container (6).

## Description

The invention relates to a soft mist inhaler or more generally to an apparatus for atomizing a drug or medication formulation. The invention more specifically relates to a metered dose nebulizer, comprising a continuously pressurized collapsible container, further comprising a high-pressure pump with metering chamber with a priming stroke and a high-pressure atomizing stroke, and an atomizer connected to the chamber outlet, wherein the collapsible container is connected to the metering chamber inlet via a liquid passageway to allow liquid flow from the collapsible container to the metering chamber during the priming stroke.

Soft Mist Inhalers (SMIs), which in more general terms may be referred to as devices for dispensing a metered quantity of fluid as a spray of droplets by discharging the metered quantity of fluid under pressure through an atomizing means, are a type of portable or pocket liquid solution or formulation metered dose inhaler. The formulation may contain a soluble drug, contain suspended drug micro-solids, suspended peptides or may be an emulsion. It can be a small molecule or a biological. They are multi-dose devices and can be mechanical with the inhalable spray produced by colliding or impinging jets, Rayleigh jets or electrically powered vibrating meshes.

In common with pressurized metered dose inhalers (pMDIs) and dry power inhalers (DPIs), SMIs are intended to produce a repeatable inhalable fine mist of solution droplets, and also to generate multiple, in particular 30 to 120, consistent delivered dose volumes or active drug masses. To achieve this, they either store the drug in a container in combination with a metering chamber (pMDIs and some DPIs) or store the individual doses separately (DPIs).

Documents US 5,497,944, US 5,662,271, and US 7,341,208 each describe a colliding jet soft mist inhaler that has significant advantages over the more conventional Dry Power Inhalers and pressurized Metered Dose Inhalers. The Inhalable Fraction or the fraction of the drug that reaches the target lungs in these devices is more than 60% and as high as 90% as against under 25% or lower for DPIs and MDIs. This potentially decreases side-effects as less total drug is delivered to the body and in addition reduces the amount of drug that needs to be produced and delivered to the environment. The inhaler disclosed there comprises a high pressure metering pump having a metering pressure chamber for containing a metered quantity of fluid at a first pressure, wherein said metering pressure chamber for the purpose of filling with said metered quantity of fluid may be put in fluid communication with a collapsible container containing said fluid, further comprising means to subject said metered quantity of fluid to a predetermined increase in pressure from said first pressure to a second pressure, and an atomizer that may be put in fluid communication with said metering pressure chamber.

In these documents, a high-pressure metering pump system is used with an operating pressure of typically a few hundred bars. The liquid formulation is atomized by a number of, in particular two, colliding liquid jets. The nozzle that produces the jets consists of two very small channels or orifices each of under 10 micrometers hydraulic diameter. The high-pressure metering pump consists of a piston cylinder arrangement. The drug solution is stored in a collapsible container such as a bag or a moving piston container. The drug is transferred to the high-pressure metering pump by suction while the collapsibility of the container relies on the low pressure generated by the suction in the metering pump.

One drawback of such a system may be seen in that the container might not collapse properly due to material and friction resistances resulting in an under-pressure (less than atmospheric pressure) in the container. Additionally, the non-return valve in between the high-pressure pump and container of actual devices only seals at relatively high pressures, so at rest the metering pump is in fluid communication with the collapsible container keeping the whole system at an under pressure. This may cause gas bubbles to form within the metering pump leading to under dosing. Additionally, the pressure difference between the bag and the metering pump can be insufficient to fill the metering pump chamber with a full dose during the priming stroke leading to under dosing. To overcome this very large wasteful overfills are required.

If a collapsible bag is used, material elasticity and memory prevent uniform and irreversible collapsibility. Also in plastic only bags, air can permeate via the plastic walls if there is an under pressure leading to additional air in the bag. The head space created in the bag means the device needs to be primed in the upright position if it has a dip tube within. In a moving piston container is used the piston or stopper needs a certain under pressure to overcome wall friction (glide force) and stiction (break loose force) or else vapor bubbles may form within the cartridge and the high-pressure metering pump during priming, leading to serious underdosing.

Additionally, an increasing under-pressure can build up within the cartridge and metering pump over time. Because the pump seals are permeable to air these can permeate air into the high-pressure pump in between puffs or drug doses.

Having to overfill the container to avoid these problems is not ideal and is wasteful in every way including ecologically and economically.

US 5,497,944 and US 5,662,271 describe a device with a high-pressure metering chamber incorporating a valve arrangement to control and regulate the flow into and out of the pressure chamber including:
A metering pressure chamber inlet non-return valve.
A metering pressure chamber outlet non-return valve.
A pressure-release valve at the metering pressure chamber outlet.

In this design, the pressure-release valve is optionally mounted in between the metering pressure chamber outlet and the atomizer or nozzle with the purpose of reducing any premature release of liquid via the nozzle before the system reaches its operating pressure during the atomizing stroke. The opening pressure of this valve therefore is selected close to the operating pressure of the pump to effectively operate.

It is an object of the present invention to provide an improved soft mist inhaler of the kind identified above that provides high reliability in avoiding leakage via the nozzle in a particularly simple design.

According to an aspect of the present invention, for an inhaler of the type identified above this object is achieved by providing a pressure-release valve at the metering pressure chamber outlet designed for an opening pressure of about two times, in particular about 20-50%, preferably of about 30%, above the designed cartridge gauge pressure.

Within the scope of this disclosure, and in the following detailed explanation, pressure values given shall be understood as gauge pressure values, i. e. a valve having an opening pressure of 2 bars shall be understood as being designed to open at a pressure of 2 bars above atmospheric pressure (which also is approximately 1 bar).

The invention is based upon the consideration that for efficient operation of the device, the dynamics of the opening process of the pressure release valve in view of the pressure-build up in the metering pressure chamber should be taken into account. In particular, it has been found that the pressure in the metering pressure chamber builds up almost instantaneously because of the relatively small nozzle orifices in the atomizer element. Accordingly, for particularly smooth and efficient operations, the opening pressure for the pressure release valve should be defined in reference to the pressure level in the cartridge, rather than (as in previous systems) the operating pressure in the pressure chamber during the atomizing stroke.

The pressure in the cartridge is defined by the maximum friction between the cartridge piston or stopper and the cartridge inner walls and the cartridge diameter. For a standard ISO glass cartridge with an inner diameter of 9,65mm and a maximum break loose force of 6N, this translates into a gauge pressure of approximately 1 ,5bars. As a consequence, in accordance with one aspect of the invention, the opening gauge pressure of the pressure release valve may be chosen at around 3 bars, preferably in an interval between 2 bars and 5 bars. In consequence, the designated opening pressure in accordance with one aspect of the invention is about a factor of more than 10 lower than that of previous systems (there: typically just under 250 bars), allowing for fundamentally different design and materials choice of surrounding components like seals etc.

In other words, in accordance with aspects of the present invention, a metered dose nebulizer is provided, comprising a continuously pressurized collapsible container, optionally further comprising either a barrel and a piston or plunger or a collapsible flexible bag containing a liquid formulation, yet further comprising a high-pressure pump with metering chamber with a priming stroke and a high-pressure atomizing stroke, and an atomizer connected at the chamber outlet. The collapsible container may be connected to the metering chamber inlet via a liquid passageway to allow liquid flow from the collapsible container to the metering chamber during the priming stroke, and a non-return valve may be located in the liquid pathway to prevent backflow from the metering chamber back to the pressurized collapsible container during the atomizing stroke. Further, in an aspect of the present invention, a pressure-relief valve is located in between the metering chamber and the atomizer to prevent liquid contents in the pressurized collapsible container reaching the atomizer and leaking under the pressure in the pressurized collapsible container, wherein the pressure-relief valve opens under the high pressure in the metering chamber during the atomizing stroke, atomizing the liquid contents of the metering chamber in the atomizer, and wherein the pressure-relief valve opens at a pressure not much greater the cartridge maximum pressure to minimize fluidic friction losses during the atomizing stroke.

During the atomizing stroke in a colliding or impinging jet SMI, the cartridge pressure is generally less than 5% of the operating pressure. Accordingly, in the present invention the opening pressure of the pressure-release valve of the present invention is much lower than the opening pressure of the pressure-release valve of previous systems.

In a preferred embodiment, the opening pressure of the pressure-relief valve is at least 20% higher than the gauge pressure in the container.

Preferably the pressure-relief valve has one or two moving parts and is preferably made from the same materials as the high-pressure pump metering chamber and seals.

In an aspect of the invention, the liquid pathway may be a capillary pathway. The liquid pathway may be a capillary pathway formed inside the pump piston.

In further aspects of the invention the collapsible container is an ISO glass cartridge or plastic including ISO COP or COC plastic cartridges with an elastomeric stopper or piston. The cartridge piston is biased by a pressurizing biased means. The biased means may be permanent or liquefied gas. The biased means may be a coiled spring or nested coiled springs to minimize the total spring length and minimize force changes as the springs expand.

The maximum force generated on the cartridge piston by the biased means preferably is selected appropriately to be easily sufficient to overcome the initial friction (break loose force) between the piston or stopper and the cartridge inner walls. The force generated on the cartridge piston by the biased means in particular should be easily sufficient to overcome the moving friction (glide force) between the piston or stopper and the cartridge inner walls.

The pressure-relief valve preferably is designed to only open at a pressure higher than the cartridge pressure. A safety margin between the opening valve pressure and the cartridge pressure preferably is sufficient to always ensure the valve doesn't open before the high-pressure pump is activated. The opening pressure of the pressure-release valve should preferably be small enough compared to the high-pressure pump operating pressure so that it has low fluidic losses and has little effect on the liquid pressure entering the atomizer nozzle.

In the case of the present invention the opening pressure of the pressure-release valve may be between 2 bars and5 bars while the high-pressure pump operating pressure may be more than 100bars or more than 200bars.

In the case of Rayleigh jet SMIs the opening pressure of the pressure-release valve would be dependent on the container type and the collapsibility generated friction of said container while the high-pressure pump operating pressure is generally not more than 50bars.

In the present invention the pressure in the collapsible container is preferably arranged to be less than 10 bars and at least enough to overcome any collapsibility resistance of the container. The pressure in the high-pressure pump and metering chamber is arranged to be more than 50 bars and in practice over 100bars or more. When used for other purposes such as nasal delivery the pressure can be less as the particles size required is greater and less energy is required for atomization.

The present invention in preferred embodiments may be a metered multi-dose inhaler or a metered multi-dose nasal or ophthalmic delivery system or any other metered dose atomization or nebulization delivery system. Particle sizes and spray velocities can be adjusted according to use by changing the atomization pressure and/or the atomizer physical characteristics. Metered dose volumes can vary between under 5 microliters to over 200 microliters.

In favor of a particularly simple and efficient design, in one aspect of the invention the pressure release valve may be formed of a membrane having a slit, wherein the opening pressure of the pressure-relief valve is a function of the membrane material, thickness and the diameter or cross-sectional area of a liquid passageway in the valve. In an embodiment preferred even further, the opening pressure of the pressure-relief valve may also be a function of the length of the slot.

The metered dose nebulizer may be re-usable and used with more than one collapsible container.

The invention may be used with any drug formulation or combination and may contain a soluble drug, contain suspended drug micro-solids, suspended peptides or may be an emulsion. It can be a small molecule or a biological drug.

The atomizer may be a mechanical beak up atomizer such as a double jet impinger, colliding or impinging jets or even a swirl chamber type atomizer or any other atomizer. The atomizer may include within a filter to minimize blockage of the small exit holes. There may be a pre-filter located before the atomizer.

The present invention is a big improvement to the prior art much improving the performance and/or simplifying the nebulizer over the prior art. The invention eliminates mechanical complexity leading to a more reliable SMI with much improved patient usability such as 360 degrees priming, large number of doses, little or no overfill and reduces considerably user misuse such as accessing the required overfill of the container with the consequential under dosing of the drug to the patient, which is common in some markets.

Preferred embodiments and aspects of the invention are described further in connection with a drawing. In this drawing,
- Fig. 1: shows a soft mist inhaler at rest,
- Fig. 2: shows the soft mist inhaler of Fig. 1 during a high-pressure atomizing stroke, and
- Fig. 3: shows a pressure-relief valve

Identical parts are labelled with the same reference numerals.

In the Figs., the soft mist inhaler 1 is shown schematically only, and external components such as the outer casing and mouthpiece are not shown; only the inner parts and the fluidic mechanisms are shown. Generally, the device 1 is intended to be manually operated by the user. To load or cock the device 1 and compress the spring 2, the user for instance may twist the lower and upper cases relative to one another. The spring 2 may be held by a latch released by a push button. The spring 2 is preferably pre-compressed to minimize spring force changes in operation.

The metered dose nebulizer 1 or soft mist inhaler 1 comprises a high-pressure metering pump 4 attached to a collapsible container 6. For simplicity, in the embodiment shown the collapsible container 6 is an ISO glass or plastic cartridge 8 with a piston or stopper 10 with liquid contents 12 within. Instead, it might also comprise a collapsible bag containing the liquid contents 12. The high-pressure metering pump 4 comprises a cylinder body 14 with a metering pressure chamber or cavity 16 within, with an atomizer 18 mounted at one end.

A piston 20 is mounted within the metering chamber 16 of the cylinder 14. The metering chamber 16 is in fluid communication with the cartridge contents 12 via a capillary pathway 22. A non-return valve 24 is mounted at the end of the capillary pathway 22 within the metering chamber 16 that allows liquid 12 to flow into the pressure metering chamber 16 when the high-pressure metering pump 4 is primed during the priming stroke but prevents backflow into the collapsible container 6 when the high-pressure metering pump 4 compresses its contents within metering chamber 16 during the atomizing stroke. A seal 26 prevents leakage from the pump between the cylinder 14 and the metering chamber 16 and the piston 20. Piston 20 is rigidly connected to the container 6 at a position 28, and the container 6 moves with the piston 20 to, during the priming stroke, fill the metering chamber 16 and, during the atomizing stroke, pressurize and atomize the liquid contents of the metering chamber 16 into a fine mist.

To prime the high-pressure metering pump 4, the piston 20 is moved relative to the cylinder body 14 in a retracting move to fill the pressure metering chamber 16. The retraction may be effected due to the rigid connection of piston 20 to the container 6, e. g. by pulling the container 6 in outward direction away from cylinder body 14. During this piston movement in a direction out of the cylinder body 14, a low pressure is generated within the pressure metering chamber 16 sufficient to suck liquid 12 from the collapsible container 6 into the pressure metering chamber 16 and either to collapse a bag (embodiment not shown) or to move the stopper 10. In the embodiment shown and in accordance with one aspect of the invention, this movement of the stopper 10 is supported by compressed spring 2 which exerts a continuous force on the stopper 10, thereby also pressurizing contents 12.

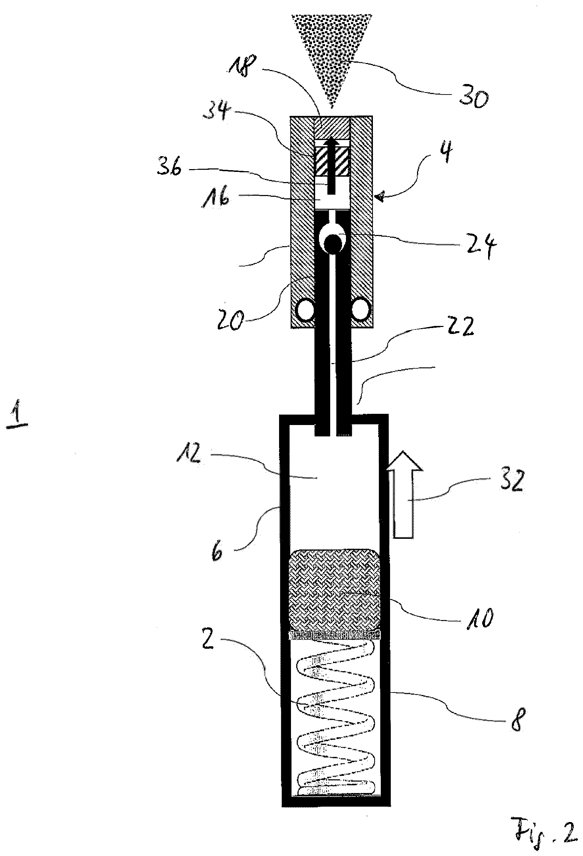

After the priming stroke, the actual atomizing stroke may be initiated. In Fig. 2, the soft mist inhaler 1 is shown during this atomizing stroke during which an aerosol cloud 30 or soft mist atomized drug solution is produced. The container 6 and, together with it due to their rigid connection, the piston 10 are moved in the direction of arrow 32, i. e. towards the cylinder body 14. As a consequence, the volume of the metering pressure chamber 16 is reduced, resulting in a significant pressure increase within the metering pressure chamber 16. During this period, the non-return valve 24 is closed to prevent back-flow from the pressure metering chamber 16 into the capillary pathway 22 and back into the collapsible container 6. The movement of the piston 20 thus forces liquid 12 in the pressure metering chamber 16 through the atomizer 18, thereby atomizing the liquid and creating the inhalable aerosol cloud 30.

In the embodiments shown, the piston 20 is driven by an external spring mechanism not shown in the Figures. Likewise, the whole mechanism comprising the cylinder 14, atomizer 18, piston 20, and container 6 are supplied within an external casing with a mechanism to prime and operate the pump 4. Further, in general the atomizer 18 may be equipped with an in-built filter, and there may also be an additional pre-filter to prevent blockage of the atomizer 18.

In accordance with aspects of the present invention, the soft mist inhaler 1 is designed to provide high reliability in avoiding leakage via the atomizer 18 in a particularly simple and cost-efficient design. In view of this purpose, the soft mist inhaler 1 in an aspect of the invention is designed for particularly efficient pressure management during the different phases identified above. In view of this design goal, the pressure in the various chambers should be managed such that leakage is minimized whereas efficient generation of the inhalable aerosol cloud 30 by appropriate use of the pressure levels in the metering pump 4 and cartridge 8 during the respective phases of operation is achieved.

In an aspect of the present invention, this object is achieved by a pressure-relief valve 34 at the outlet of the metering pressure chamber 16, before the atomizer 18. Regarding its characteristics, in particular its opening pressure, the pressure-relief valve in accordance with aspects of the present invention is designed under the consideration that for efficient operation of the inhaler 1, the dynamics of the opening process of the pressure release valve in view of the pressure-build up in the metering pressure chamber 16 should be taken into account. In particular, in view of the surprising recognition that the pressure in the metering pressure chamber 16, upon activation of the atomizing stroke, builds up almost instantaneously to a comparatively high level, as compared to typical device pressures (in the container 6 or in the metering pressure chamber 16), a relatively quick response of the pressure-relief valve 34 in reaction to pressure build-up in the metering pressure chamber 16 is considered preferable. Accordingly, under aspects of avoiding leakage from the container 6 in between activations or when the device is not being used and for particularly smooth and efficient operations with quick reaction times, the pressure-relief valve 34 is designed in reference and in comparison to the pressure level in the cartridge 6, rather than (as in previous systems) the operating pressure in the pressure chamber 16 during the atomizing stroke.

As a consequence, in accordance with one aspect of the invention, the pressure release valve 34 is designed for an opening pressure of about 30%, or, more generally speaking, in the interval of 20-50%, above the designed cartridge pressure. In view of typical systems, this means that the opening pressure of the pressure-relief valve 34 may be chosen at around 3 bars, preferably in an interval between 2 bars and 5 bars. In consequence, in comparison to other systems of similar type, the designated opening pressure in accordance with one aspect of the invention is about a factor of more than 10 lower than that of previous systems (there: typically just below the operating pressure of 250 bars), allowing for fundamentally different design and materials choice of surrounding components like seals etc. Additionally such a low opening pressure relative to the operating pressure minimizes fluidic losses during the atomizing stroke compared to high fluidic losses in the opening valve 34 when the pressure is close to the operating pressure, as in previous systems.

The pressure-relief valve 34, among other effects, allows the collapsible container 61 to be at a constant pressure to aid priming without leakage of the cartridge contents 12 out via atomizer 18. It avoids any intermittent pressurization of cartridge contents 12 or control of the flow of cartridge contents 12 by complex mechanical arrangements as deemed necessary in other, similar systems. In particular, due to its specific characteristics, the pressure release valve 34 opens at the beginning of the high-pressure atomization stroke, and at a pressure a little over the container pressure.

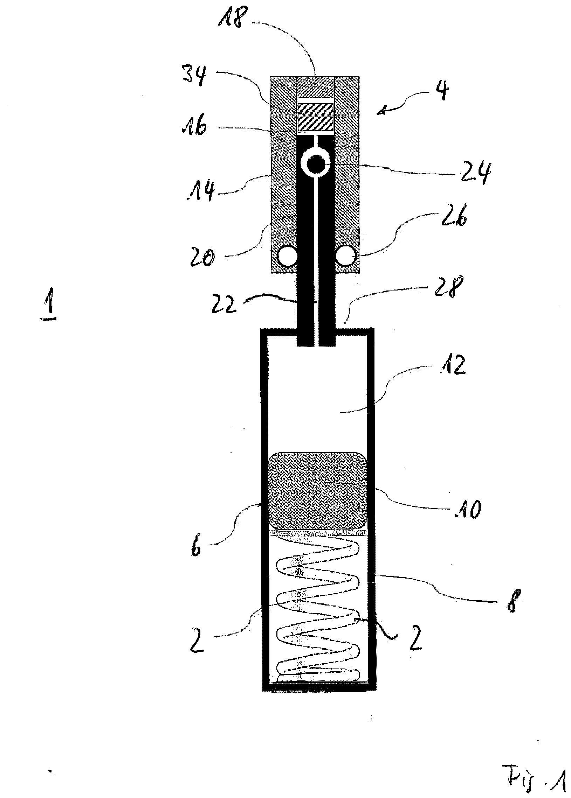

More precisely, in Fig. 1 the soft mist inhaler 1 is at rest. As mentioned, the high-pressure metering pump 4 comprises the pressure-relief valve 34 placed in between the pressure metering chamber 16 and the atomizer 18. The liquid contents 12 are under pressure from the force of the spring 2 on the stopper 10 of the container 6. The liquid in the metering pressure chamber 16 is also under pressure generated by the spring 2 as the non-return valve 24 is open. The pressure-relief valve 34, in accordance with the considerations explained above, is arranged to be closed at the pressure level generated by the spring 2 which is much lower than the pressure generated by the high-pressure metering chamber 16 during the atomization stroke.

Figure 2, on the other hand, shows the soft mist inhaler 1 during the high-pressure atomizing stroke. The container 6 and piston 20 together with stopper 10 are moving in the direction of arrow 32, generating a high pressure within the metering chamber 16 of the cylinder body 14 of the high-pressure metering pump 4. In this phase of operation, the non-return valve 24 is closed to prevent back-flow via the capillary pathway 22. The valve 34 is open due to the high pressure in the metering chamber 16, forcing liquid through as shown by arrow 36, and through atomizer 18 to form the aerosol cloud 30.

During operation of the soft mist inhaler 1 of the embodiment shown, some losses in the pressure relief valve 34 may be expected. As an example of the present embodiment, the operating pressure during the atomizing stroke may be 250 bars. The cartridge 6 may have a design pressure at a maximum of 2 bars, which is deemed sufficient to overcome piston friction. Accordingly, during the atomizing stroke the cartridge pressure is less than 2% of the operating pressure. The opening pressure of the pressure-relief valve 34 thus may be 3 bars. The losses in a simple valve design are estimated by simplified calculation to be to be 3/250 or 1,5%, which in accordance with one aspect of the invention is considered relatively low and thus superior to similar systems.

The maximum force generated on the cartridge piston or stopper 10 by the biased spring 2 preferably is selected appropriately to be easily sufficient to overcome the initial friction (break loose force) between the piston or stopper 10 and the inner walls of cartridge 6. The high pressure in the metering chamber 16 may be generated by a 45N mean force spring without the pressure-relief valve. Depending on the fluid to be atomized this may have to be increased by about 2% to maintain particle size and distribution to about 46N with a subsequent small increase in the manual cocking force of the device.

The container 6 is preferably inserted into the device 1 only after the device 1 has been removed from its packaging and after storage which may be up to 2 years. Consequentially, valve 34 only operates during use of the device 1 which typically will be 1 month or between 1 week and 2 months depending on number of doses to be delivered by the device 1.

The device 1 maybe used with one or more cartridges 8.

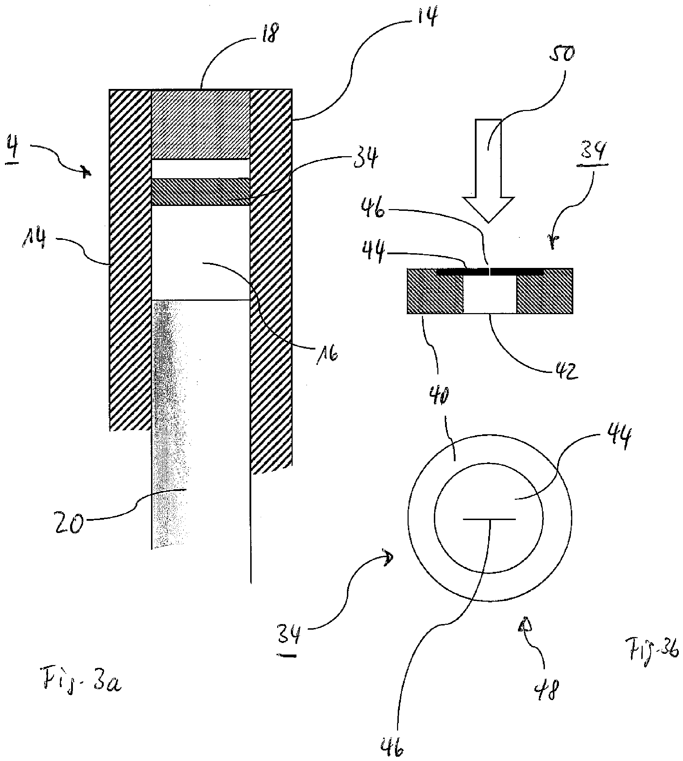

Figures 3 shows the pressure-relief valve 43 according to the invention in further detail.

In Figure 3a, the high-pressure metering pump 4 comprises a cylinder body 14 with a metering pressure chamber or cavity 16 within, with an atomizer 18 mounted at one end, is shown in enlarged view. As described above, the piston 20 is mounted within the metering chamber 16 of the cylinder 14. The pressure-relief valve 34 is placed within the cylinder body 14 in between piston 20 and atomizer 18.

Figure 3b shows further detail of the pressure release valve 34. A valve housing 40 has a passageway 42 within and rigidly holds a flexible membrane 44 which has a cut or liquid passageway 46. This passageway 46 is closed to liquid until the pressure in the metering chamber 16 exceeds a certain predefined level. View 48 shows the valve 34 as indicated by arrow 50.

The opening pressure of the pressure-relief valve 34 according to one aspect of the invention is a function of the membrane 44 material, thickness and the diameter or cross-sectional area of liquid passageway 42 assuming slot 46 is the same length as the diameter of passageway 42. In a preferred embodiment and in a typical application, for an opening pressure of 3bars and for silicone rubber as selected material for the membrane 44, this corresponds to a membrane 44 thickness between 0,1mm to 0,3mm and a passageway 42 diameter of between 0,5mm and 2mm.

Other valve designs and dimensions are possible according to the invention.

### List of reference numerals

- 1: soft mist inhaler
- 2: spring
- 4: high pressure metering pump
- 6: container
- 8: cartridge
- 10: stopper
- 12: liquid contents
- 14: cylinder body
- 16: metering pressure chamber
- 18: atomizer
- 20: piston
- 22: capillary pathway
- 24: non-return valve
- 26: seal
- 28: position
- 30: cloud
- 32: arrow
- 34: pressure-relief valve
- 36: arrow
- 40: housing
- 42: passageway
- 44: membrane
- 46: cut or passageway
- 48: view
- 50: arrow

## Claims

1. Soft mist inhaler (1), comprising a continuously pressurized collapsible container (6), a high-pressure pump (4) with a metering chamber (16), and an atomizer (18) connected to the outlet of the metering chamber (16), wherein the collapsible container (6) is connected to an inlet of the metering chamber (16) via a liquid passageway (22) to allow liquid flow from the collapsible container (6) to the metering chamber (16) during a priming stroke, **characterized by** a pressure-relief valve (34) provided in the connection between the metering chamber (16) and the atomizer (18), having an opening pressure of not more than twice, preferably not more than 150% of, the gauge pressure in the container (6).

2. Soft mist inhaler (1) according to claim 1, in which the opening pressure of the pressure-relief valve (34) is at least 20% higher than the gauge pressure in the container (6).

3. Soft mist inhaler (1) according to claim 1 or 2, in which the pressure release valve (34) is formed of a membrane (44) having a slit (46), wherein the opening pressure of the pressure-relief valve (34) is a function of the membrane (44) material, thickness and the diameter or cross-sectional area of a liquid passageway (42) in the valve (34).

4. Soft mist inhaler (1) according to claim 3 wherein the opening pressure of the pressure-relief valve (34) is a function of the length of the slit (46).
